# EUROPEAN PATENT APPLICATION

(11) **EP 2 415 863 A1**
(43) Date of publication of application: **08.02.2012**
(21) Application number: 10008094.4
(22) Date of filing: 03.08.2010
(51) Int. Cl.: C12N 9/04

(54) **Mutant glucose oxidase**

(71) Applicant: B.R.A.I.N., 64673 Zwingenberg (DE)
(72) Inventor: The designation of the inventor has not yet been filed
(74) Representative: Vossius & Partner

(57) **Abstract**

The present invention relates to a novel nucleic acid molecule encoding (I) a polypeptide having the activity of a glucose oxidase (EC 1.1.3.4); or (II) a fragment of the polypeptide of (I) having the activity of a glucose oxidase and comprising at least 400 amino acids. Moreover, the invention relates to a vector encoding the nucleic acid molecule, a host cell transformed, transduced or transfected with the vector, a the protein encoded by the nucleic acid molecule, and compositions comprising said nucleic acid molecule, vector, host cell, protein or combinations thereof.

## Description

The present invention relates to a nucleic acid molecule encoding (I) a polypeptide having the activity of a glucose oxidase (EC 1.1.3.4), which is (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule encoding a the glucose oxidase the amino acid sequence of which is at least 85 %, preferably at least 90 % identical, more preferably at least 95 % identical, and most preferred at least 98% identical to the amino acid sequence of SEQ ID NO: 1, provided that at least three of (i) to (v) apply: (i) tyrosine, serine, threonine, cysteine, asparagine, glutamine, aspartic acid or glutamic acid is present in the amino acid position corresponding to amino acid position 2 of SEQ ID NO: 1, (ii) glutamic acid, aspartic acid, tyrosine, serine, threonine, asparagine or glutamine is present in the amino acid position corresponding to amino acid position 13 of SEQ ID NO: 1, (iii) valine, glycine, alanine, isoleucine, leucine, methionine, phenylalanine, tryptophan or proline is present in the amino acid position corresponding to amino acid position 30 of SEQ ID NO: 1, (iv) valine, glycine, alanine, isoleucine, leucine, methionine, phenylalanine, tryptophan or proline is present in the amino acid position corresponding to amino acid position 94 of SEQ ID NO: 1, and (v) arginine or histidine is present in the amino acid position corresponding to amino acid position 152 of SEQ ID NO: 1; (d) a nucleic acid molecule comprising or consisting of a nucleotide sequence which is at least 85 % identical, preferably at least 90 % identical, more preferably at least 95 % identical, and most preferred at least 98 % identical to the nucleotide sequence of SEQ ID NO: 2; provided that at least three of (i) to (v) apply: (i) nucleotide positions 4 to 6 of SEQ ID NO: 2 code for tyrosine, serine, threonine, cysteine, asparagine, glutamine, aspartic acid or glutamic acid, (ii) nucleotide positions 26 to 28 of SEQ ID NO: 2 code for glutamic acid, aspartic acid, tyrosine, serine, threonine, asparagine or glutamine, (iii) nucleotide positions 90 to 92 of SEQ ID NO: 2 code for valine, glycine, alanine, isoleucine, leucine, methionine, phenylalanine, tryptophan or proline, (iv) nucleotide positions 282 to 284 of SEQ ID NO: 2 code for valine, glycine, alanine, isoleucine, leucine, methionine, phenylalanine, tryptophan or proline, and (v) nucleotide positions 456 to 458 of SEQ ID NO: 2 code for arginine or histidine; (e) a nucleic acid molecule which is degenerate with respect to the nucleic acid molecule of (d); or (f) a nucleic acid molecule corresponding to the nucleic acid molecule of any one of (a) to (e) wherein T is replaced by U; (II) a fragment of the polypeptide of (I) having the activity of a glucose oxidase and comprising at least 400 amino acids, provided that at least three of (i) to (v) apply (i) tyrosine, serine, threonine, cysteine, asparagine, glutamine, aspartic acid or glutamic acid is present in the amino acid position corresponding to amino acid position 2 of SEQ ID NO: 1, (ii) glutamic acid, aspartic acid, tyrosine, serine, threonine, asparagine or glutamine is present in the amino acid position corresponding to amino acid position 13 of SEQ ID NO: 1, (iii) valine, glycine, alanine, isoleucine, leucine, methionine, phenylalanine, tryptophan or proline is present in the amino acid position corresponding to amino acid position 30 of SEQ ID NO: 1, (iv) valine, glycine, alanine, isoleucine, leucine, methionine, phenylalanine, tryptophan or proline is present in the amino acid position corresponding to amino acid position 94 of SEQ ID NO: 1, and (v) arginine or histidine is present in the amino acid position corresponding to amino acid position 152 of SEQ ID NO: 1.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Glucose oxidases (E. C. 1.1.3.4) are enzymes that catalyze the oxidation of glucose with oxygen, such that D-gluconic acid and hydrogen peroxide are formed by the following chemical reaction :

D-Glucose+O₂ → Gluconolactone+H₂O₂

Glucose oxidases (GOx) from different microbial origins have been described. W089/126675 describes the production of glucose oxidase from *Aspergillus niger* in recombinant systems and WO 2008/079227 A1 relates to a glucose oxidase obtained from *Aspergillus niger* formulated in a composition conferring improved storage stability. Glucose oxidases from different origins were also disclosed such as GOx form marine algae, e. g. *Chondrus crispus* (US 7544795, US 6924366), filamentous fungi, e. g. *Cladosporium sp.* (WO 95/29996, WO 1998/020136, US 5834280) or *Talaromyces flavus* (US 6054318).

These enzymes are of considerable commercial importance since they have numerous uses e.g. in the food industry, and in the medical sector. They are commonly used in biosensors for the detection of glucose in industrial solutions and body fluids, e.g. blood and urine. A main application might become the use of glucose oxidases in the anodic compartment of implanted and miniaturized biofuel cells burning glucose from the blood stream and thereby powering miniature diagnostic devices or pumps. The use of GOx in biosensors is of significant interest. E. g. WO 2009/104836 A1 describes a glucose sensor comprising a genetically engineered glucose oxidase variant improved for its attachment to metal surfaces. Furthermore, a use of glucose oxidase is suggested in the production of transgenic plants and other organisms with reduced susceptibility or increased resistance to pests or diseases (WO 1995/021924 A1).

Applications in the food industry are numerous, since the capability of oxygen oxidoreductases including that of glucose oxidase to generate hydrogen peroxide, which has an antimicrobial effect, can be utilized to improve the storage stability of certain food products including cheese, butter and fruit juice. It has also been suggested that oxidoreductases may be potentially useful as oxygen scavengers or antioxidants in food products. For example U.S. Pat. No. 2,783,150 and WO 2009/130306 A1 disclose the addition of glucose oxidase to flour to improve the characteristics of dough, CA 2,012,723 discloses bread improving agents comprising cellulolytic enzymes and glucose oxidase.

Applications of glucose oxidase in cosmetic compositions may utilize the antimicrobial properties of the peroxide generated by the enzyme and the antioxidant properties of GOx. Potential uses for hexose oxidases in pharmaceutical and cosmetic compositions are suggested e.g in US 6924366, US 6251626 and WO 2007/ 045251 A2.

As it is evident from the above, glucose oxidase is an enzyme having many important applications in industry as well as in medicine. Accordingly, there is an ongoing need for further glucose oxidases, ideally being improved as compared to the glucose oxidases known from the prior art. This need is addressed by the present invention.

Accordingly the present invention relates in a first embodiment to a nucleic acid molecule encoding (I) a polypeptide having the activity of a glucose oxidase (EC 1.1.3.4), which is (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule encoding a glucose oxidase the amino acid sequence of which is at least 85 %, preferably at least 90 % identical, more preferably at least 95 % identical, and most preferred at least 98% identical to the amino acid sequence of SEQ ID NO: 1, provided that at least three of (i) to (v) apply: (i) tyrosine, serine, threonine, cysteine, asparagine, glutamine, aspartic acid or glutamic acid is present in the amino acid position corresponding to amino acid position 2 of SEQ ID NO: 1, (ii) glutamic acid, aspartic acid, tyrosine, serine, threonine, asparagine or glutamine is present in the amino acid position corresponding to amino acid position 13 of SEQ ID NO: 1, (iii) valine, glycine, alanine, isoleucine, leucine, methionine, phenylalanine, tryptophan or proline is present in the amino acid position corresponding to amino acid position 30 of SEQ ID NO: 1, (iv) valine, glycine, alanine, isoleucine, leucine, methionine, phenylalanine, tryptophan or proline is present in the amino acid position corresponding to amino acid position 94 of SEQ ID NO: 1, and (v) arginine or histidine is present in the amino acid position corresponding to amino acid position 152 of SEQ ID NO: 1; (d) a nucleic acid molecule comprising or consisting of a nucleotide sequence which is at least 85 % identical, preferably at least 90 % identical, more preferably at least 95 % identical, and most preferred at least 98 % identical to the nucleotide sequence of SEQ ID NO: 2; provided that at least three of (i) to (v) apply: (i) nucleotide positions 4 to 6 of SEQ ID NO: 2 code for tyrosine, serine, threonine, cysteine, asparagine, glutamine, aspartic acid or glutamic acid, (ii) nucleotide positions 26 to 28 of SEQ ID NO: 2 code for glutamic acid, aspartic acid, tyrosine, serine, threonine, asparagine or glutamine, (iii) nucleotide positions 90 to 92 of SEQ ID NO: 2 code for valine, glycine, alanine, isoleucine, leucine, methionine, phenylalanine, tryptophan or proline, (iv) nucleotide positions 282 to 284 of SEQ ID NO: 2 code for valine, glycine, alanine, isoleucine, leucine, methionine, phenylalanine, tryptophan or proline, and (v) nucleotide positions 456 to 458 of SEQ ID NO: 2 code for arginine or histidine; (e) a nucleic acid molecule which is degenerate with respect to the nucleic acid molecule of (d); or (f) a nucleic acid molecule corresponding to the nucleic acid molecule of any one of (a) to (e) wherein T is replaced by U; (II) a fragment of the polypeptide of (I) having the activity of a glucose oxidase and comprising at least 400 amino acids, provided that at least three of (i) to (v) apply (i) tyrosine, serine, threonine, cysteine, asparagine, glutamine, aspartic acid or glutamic acid is present in the amino acid position corresponding to amino acid position 2 of SEQ ID NO: 1, (ii) glutamic acid, aspartic acid, tyrosine, serine, threonine, asparagine or glutamine is present in the amino acid position corresponding to amino acid position 13 of SEQ ID NO: 1, (iii) valine, glycine, alanine, isoleucine, leucine, methionine, phenylalanine, tryptophan or proline is present in the amino acid position corresponding to amino acid position 30 of SEQ ID NO: 1, (iv) valine, glycine, alanine, isoleucine, leucine, methionine, phenylalanine, tryptophan or proline is present in the amino acid position corresponding to amino acid position 94 of SEQ ID NO: 1, and (v) arginine or histidine is present in the amino acid position corresponding to amino acid position 152 of SEQ ID NO: 1.

The term "nucleic acid molecule" in accordance with the present invention includes DNA, such as _{C}DNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and doublestranded hybrids molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thioguanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The term "enzyme" in accordance with the invention means any substance composed wholly or largely of protein or polypeptides that catalyzes or promotes, more or less specifically, one or more chemical or biochemical reactions. The term "enzyme" can also refer to a catalytic polynucleotide (e.g. RNA or DNA).

The term "oxidation reaction", means in general terms a chemical or biochemical reaction involving the addition of oxygen to a substrate, to form an oxygenated or oxidized substrate or product. An oxidation reaction is typically accompanied by a reduction reaction (hence the term "redox" reaction, for oxidation and reduction). A compound is "oxidized" when it receives oxygen or loses electrons. A compound is "reduced" when it loses oxygen or gains electrons. GOx typically catalyzes the oxidation of a primary alcohol group to an aldehyde.

The term "glucose oxidase" (GOx) (E.C. 1.1.3.4.) specifies a protein that catalyzes the oxidation of beta-D-glucose into D-glucono-1,5-lactone (D-Glucose+O₂ **→** Gluconolactone+H₂O₂), which then may hydrolyze to gluconic acid. Accordingly, the glucose oxidase is an enzyme. Further, the term "a polypeptide having the activity of a glucose oxidase" refers to a poylpeptide having the afore mentioned activity.

The enzyme glucose oxidase is thus a member of the class of oxidation enzymes which catalyzes an oxidation reaction, by adding, inserting, contributing or transferring oxygen from a source or donor to a substrate. Such enzymes are also called oxidoreductases or redox enzymes, and encompass oxygenases, hydrogenases or reductases, oxidases and peroxidases. In this regard, the terms "oxygen donor", "oxidizing agent" and "oxidant" mean a substance, molecule or compound which donates oxygen to a substrate in an oxidation reaction. Typically, the oxygen donor is reduced (accepts electrons). Exemplary oxygen donors, which are not limiting, include molecular oxygen or dioxygen (O₂) and peroxides, including alkyl peroxides such as t-butyl peroxide, and most preferably hydrogen peroxide (H₂O₂). A peroxide is any compound having two oxygen atoms bound to each other.

As mentioned the "activity" of a glucose oxidase as used herein is directed to a measure of its ability to catalyze the oxidation reaction D-Glucose+O₂ → Gluconolactone+H₂O₂, and may be expressed as the rate at which the product of the reaction is produced. For example glucose oxidase activity can be represented as the amount of product (Gluconolactone and/or H₂O₂) produced per unit of time, or per unit (e.g. concentration or weight) of glucose oxidase.

The nucleic acid molecule according to the invention encodes a polypeptide or fragment thereof which is derived from glucose oxidase (GOx) from *Aspergillus niger* (SEQ ID NO: 3) was used for directed evolution to improve the kinetic properties of the enzyme in the reaction from Glucose to gluconolactone and peroxide. GOx from *Aspergillus niger* is a well-characterized protein forming a dimer, 160 kDa in size and crystal structures have been solved [Hecht, H.J., et al., Crystal-Structure Of Glucose-Oxidase From Aspergillus-Niger Refined At 2.3 Angstrom Resolution. Journal Of Molecular Biology, 1993. 229(1): p. 153-172].

Directed evolution is a powerful algorithm which allows improving enzyme properties in iterative cycle of diversity generation and screening [Matsuura, T. and T. Yomo, In vitro evolution of proteins. Journal of Bioscience and Bioengineering, 2006. 101(6): p. 449-456 et al]. Mutational loads in directed evolution experiments are often limited by the throughput of the employed screening system. For instance, in a recent review directed evolution campaigns employing monooxygenases were summarized and compared in screened library sizes and employed mutagenic conditions [Tee, K.L. and U. Schwaneberg, Directed evolution of oxygenases: Screening systems, success stories and challenges. Combinatorial Chemistry & High Throughput Screening, 2007. 10 (3): p. 197-217]. Only small library sizes (up to 10000 variants per round of evolution) with low mutagenic loads were used yielding on average one to two amino acid substitutions per round of evolution. The latter evolution strategy do not permit with decent probabilities to find synergistic mutations like two simultaneous exchanges, two oppositely charged amino acids in a loop, to increase thermal resistance or to improve two properties at once. Throughputs from 10⁵ to 10⁶ allow high mutational loads in mutant libraries and to explore novel directed evolution strategies which might "accelerate" directed protein evolution.

State of the art throughput technologies based on tyramide are microbead surface display [S Sepp, A., D.S. Tawfik, and A.D. Griffiths, Microbead display by in vitro compartmentalisation: selection for binding using flow cytometry. FEBS Letters, 2002. 532(3): p. 455-458], E. coli surface display [Becker, S., et al., Ultrahigh-throughput screening to identify E.coli cells expressing functionally active enzymes on their surface. Chembiochem, 2007. 8(8): p. 943-949] and Yeast surface display [Lipovsek, D., et al., Selection of horseradish peroxidase variants with enhanced enantioselectivity by yeast surface display. Chemistry & Biology, 2007. 14(10): p. 1176-1185], all comprising surface display in solutions.

A directed evolution method was applied to glucose oxidase to improve resistance to peroxide as described in WO 03036255 A2 employing mutated glucose oxidase genes and subsequent screening for colonies with peroxide resistant properties.

Screening methods with a throughput of 10⁵-10⁶ allow exploring novel directed evolution strategies with elevated mutational loads generating 4-6 amino acid substitutions per protein variant (mutein). The robust tyramide surface labeling has successfully been used in such high-throughput screening systems in "activity"-diluted cell suspension (population of active variants <0.001%) improving activity and inverting enantioselectivity. Further tyramide based high-throughput screening systems are microbead based systems that were used only for selection of binding properties by FACS [Sepp, A., D.S. Tawfik, and A.D. Griffiths, Microbead display by in vitro compartmentalisation: selection for binding using flow cytometry. Febs Letters, 2002. 532(3): p. 455-4583]. Reports of coupled enzyme reactions with tyramide surface labeling chemistry comprise Esterase/HRP and 2-methyldecanoic acid p-nitro-phenyl ester carring 2,4 dinitrophenyl (DNP) and biotin as indicator groups. After performing reaction in diluted cell suspension, cells were stained with Alexa Fluor 488-labeled anti-DNP antibody and/or streptavidin R-phycoerythrin conjugate [Becker, S., et al., Single-cell high-throughput screening to identify enantioselective hydrolytic enzymes. Angewandte Chemie-International Edition, 2008. 47(27): p. 5085-5088]. Unspecific labeling becomes in coupled enzyme reaction systems a limiting factor for enrichment in contrast to reported screening systems employing only horse radish peroxidase [Antipov, E., et al., Highly L and D enantioselective variants of horseradish peroxidase discovered by an ultrahigh-throughput selection method. Proceedings of the National Academy of Sciences of the United States of America, 2008. 105(46): p. 17694-17699].

*In vitro* compartmentalization (IVC) is an ultrahigh-throughput screening method (10¹⁰ reaction compartments per ml reaction volume. Enzymatic reactions are in IVC experiments performed inside aqueous microdroplets dispersed in water-in-oil-in-water emulsions. Only a few reports and reviews exist in which IVC was combined with flow cytometry sorting [Taly, V., B.T. Kelly, and A.D. Griffiths, Droplets as microreactors for high-throughput biology. Chembiochem, 2007. 8(3): p. 263-272; Bershtein, S. and D.S. Tawfik, Advances in laboratory evolution of enzymes. Current Opinion in Chemical Biology, 2008. 12(2): p. 151-158]. A main challenge for tyramide based screening systems in solution is that dye-labeled-tyramide radicals should exclusively label tyrosine on the cell surface of the displayed enzyme variant and not of neighboring cells. The latter works well for displayed Horseradish peroxidase, but is difficult to achieve in tandem enzyme reactions in which hydrogen peroxide is generated by the targeted protein (e.g. Glucose oxidase GOx). A minor limitation is the pH value of 9 which is usually employed in decorating the E. coli cells with HRP in presence of the also expressed and displayed target protein [Becker, S., et al., Ultra-high-throughput screening based on cell-surface display and fluorescence-activated cell sorting for the identification of novel biocatalysts. Current Opinion in Biotechnology, 2004. 15(4): p. 323-329].

For the purpose of the present invention, an error prone PCR mutant library of Glucose oxidase (GOx) containing 10⁷ cells and -10'5 of different GOx variants starting from GOx from *Aspergillus niger* was screened. Mutagenic conditions of GOx mutant library were selected to generate <1 % of active GOx population in order to explore influence of high mutation frequency on GOx activity.

A S. *cerevisiae* display system was used for directed evolution of GOx which employs the robust tyramide surface labeling chemistry and which is combined with an *in vitro* compartmentalization technology for enabling coupled GOx/HRP reactions with minimized cross-talk (CoaTi) and subsequent flow cytometer sorting.

Flow cytometry was applied for sorting of yeast cells for screening for novel variants of glucose oxidase (GOx). CoaTi combines *in vitro* compartmentalization technology with the CARD reporter system which uses fluorescein tyramide labels for detection of peroxidase activity. Physical connection between cells and fluorescein tyramide radicals was achieved by compartmentalization of yeast cells inside microdroplets of single water-in-oil emulsions. After reaction cells were recovered from single emulsions and sorted by flow cytometry.

For detection of GOx activity the ABTS assay was used. 2,2'-azino-bis(3-ethylbenzthiazoline-6-sulphonic acid (ABTS) is commonly used as a substrate with hydrogen peroxide for a peroxidase generating enzyme. Its use allows the reaction kinetics of peroxidases to be followed, to indirectly follow the reaction kinetics of a hydrogen peroxide-producing enzyme or to quantify the amount of hydrogen peroxide in a sample [vanGijlswijk, R.P.M., et al., Fluorochrome-labeled tyramides: Use in immunocytochemistry and fluorescence in situ hybridization. Journal of Histochemistry & Cytochemistry, 1997. 45(3): p. 375-382].

This compound is commonly chosen as substrate because the enzyme facilitates the reaction with hydrogen peroxide, turning it into a green and soluble end-product with a maximum absorbance at 420 nm.

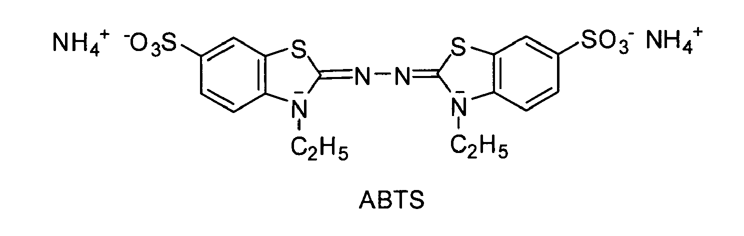

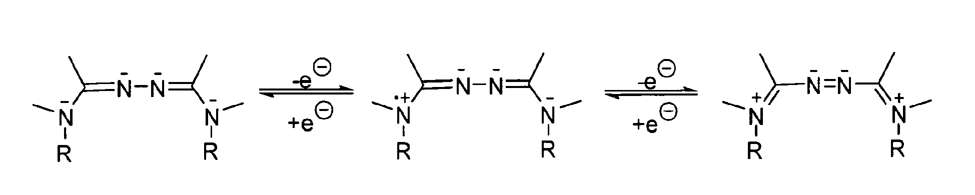

The use of ABTS as chromogen for high throughput screening of variants of galactose oxidase in microplates and on agar plates has been described (Sun L, Yagasaki M. 2003, Screen for oxidases by detection of hydrogen peroxide with horseradish peroxidise. Methods Mol Biol. 230:177-82).

The term "protein" as used herein interchangeably with the term "polypeptide" describes linear molecular chains of amino acids, including single chain proteins or their fragments, preferably containing at least 400 amino acids. Polypeptides may further form oligomers consisting of at least two identical or different molecules. The corresponding higher order structures of such multimers are, correspondingly, termed homo- or heterodimers, homo- or heterotrimers etc.. It is preferred that the polypeptides of the invention form heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is effected e.g. by glycosylation, acetylation, phosphorylation and similar modifications which are well known in the art.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence analysis and alignment in connection with the present invention was carried out using the NCBI BLAST algorithm (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) which is preferably employed in accordance with this invention. The skilled person is aware of additional suitable programs to align nucleic acid sequences.

As defined herein, sequence identities of at least 85 % identical, preferably at least 90 % identical, more preferably at least 95 % identical, and most preferred at least 98 % are envisaged by the invention. However, also envisaged by the invention are with increasing preference sequence identities of at least 98,5 %, at least 99 %, at least 99,5 %, at least 99,8 %, and 100 %.

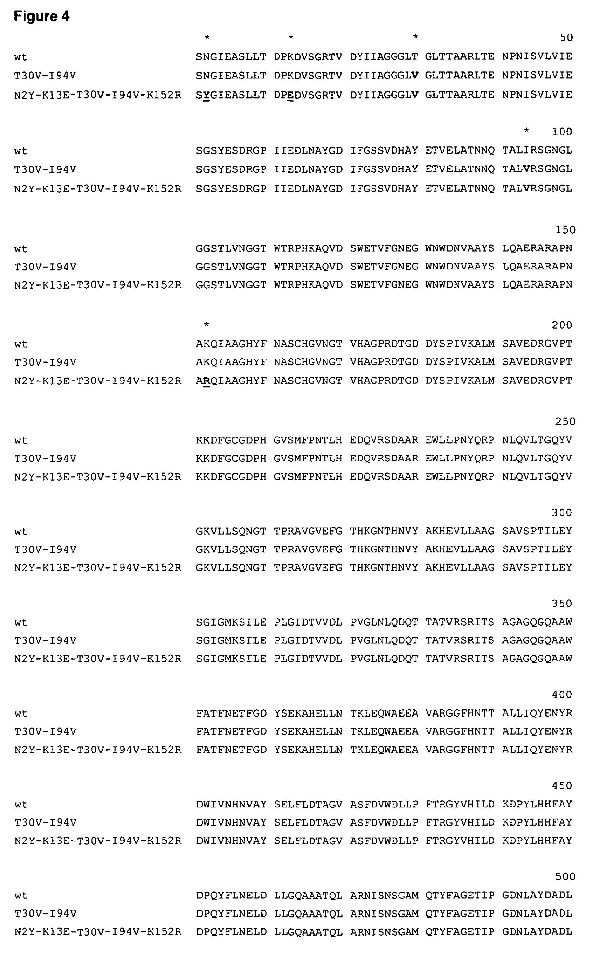

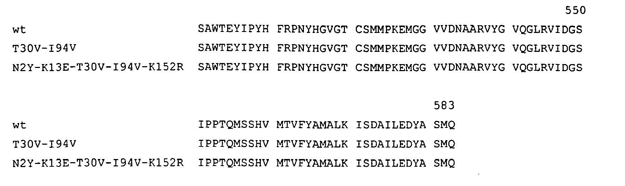

The term "provided that at least three of (i) to (v) apply" means that the nucleic acid molecule of the invention is characterized by at least three of the technical features as defined in any of (i), (ii), (iii), (iv) and (v). In this regard, it is preferred that at least four of (i) to (v) apply, and more preferred that all of (i) to (v) apply. Moreover, it is noted that irrespectively of whether (i) tyrosine, serine, threonine, cysteine, asparagine, glutamine, aspartic acid or glutamic acid is present in the amino acid position corresponding to amino acid position 2 of SEQ ID NO: 1, of whether (ii) glutamic acid, aspartic acid, tyrosine, serine, threonine, asparagine or glutamine is present in the amino acid position corresponding to amino acid position 13 of SEQ ID NO: 1, of whether (iii) valine, glycine, alanine, isoleucine, leucine, methionine, phenylalanine, tryptophan or proline is present in the amino acid position corresponding to amino acid position 30 of SEQ ID NO: 1, of whether (iv) valine, glycine, alanine, isoleucine, leucine, methionine, phenylalanine, tryptophan or proline is present in the amino acid position corresponding to amino acid position 94 of SEQ ID NO: 1, or of whether (v) arginine or histidine is present in the amino acid position corresponding to amino acid position 152 of SEQ ID NO: 1, it is expected that the polypeptide of the invention has highly similar properties. In more detail, the amino acids in (i) to (v), substituting for the amino acid in the corresponding wild-type GOx (cf. Figure 4), all have similar chemical properties, respectively, e.g. with regard to charge, polarity or hydrophobicity.

The term "degenerate" in accordance with the present invention refers to the degeneracy of the genetic code. Degeneracy results because a triplet code designates 20 amino acids and a stop codon. Because four bases exist which are utilized to encode genetic information, triplet codons are required to produce at least 21 different codes. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having a different nucleotide sequence than that specified above, but still encoding the same polypeptide lie within the scope of the present invention.

Fragments according the present invention are polypeptides having the activity of a glucose oxidase as defined herein above and comprising at least 400 amino acids. In this regard, it is preferred with increasing preference that the fragments according the present invention are polypeptides of at least 450, at least 500, at least 550, at least 575, or at least 580 amino acids and most preferred that the fragment is a fragment only lacking the amino acid methionine at amino acid one of SEQ ID NO: 1. Moreover, it is required that the fragment comprises the amino residues Tyr68, Phe414 and Trp430 of SEQ NO: 1. These three residues are in direct contact with the substrate glucose of the glucose oxidase (Meyer et la. (1998), Journal of Computer-Aided Molecular Design, 12: 425-440)

As mentioned above, the nucleic acid molecule of the invention encodes a novel variant of Glucose oxidase (GOx) derived from *Aspergillus niger* obtained by directed evolution mutagenesis and subsequent sorting of GOx expressing cells by flow cytometry. As it is evident from the examples in this specification, the novel GOx mutant having SEQ ID NO: 1, being encoded by nucleic acid sequence of SEQ ID NO: 2 and which is also termed M12 herein, is superior to the corresponding wild type enzyme (SEQ ID NO: 3, encoded by SEQ ID NO: 4) and the parent mutant (SEQ ID NO: 5, encoded by SEQ ID NO: 6), which is also termed B11 herein, in that it exhibits a significantly improved enzymatic efficiency expressed as Kc/Km. In more detail, M12 showed a 1.2 times decreased Kₘ, (22.0 vs 18.1 mM) and a 2.7 fold increased !<cat (150 s⁻¹ vs 54.8 s') compared to wt GOx (SEQ ID NO: 3). Compared to the employed parent GOx (SEQ ID NO: 5) (16 mM, 80 s'), also termed B11 herein, it has slightly increased Kin, and 1.8 times increased K_{cat}; cf. Table 1.

In general, the polypeptide of the invention may also be referred to herein as being a "mutant", "mutein" or "variant", meaning that it has been made, altered, derived, or is in some way different or changed from the wild-type protein, and/or from the parent mutant. The wild-type protein comprises the natural sequence of amino acids in the polypeptide and typically includes glycosylation. The "parent" polypeptide is any polypeptide from which any other polypeptide is derived or made, using any methods, tools or techniques, and whether or not the parent is itself a native or mutant polypeptide. A parent polynucleotide is one that encodes a parent polypeptide. SEQ ID NO. 3 of the invention differs from the mutant or variant having SEQ ID NO: 1 by the following 5 amino acid mutations (substitutions): N2Y, K13E, T30V, 194V, and K152R. Moreover, SEQ ID NO. 6 of the invention differs from the mutant or variant having SEQ ID NO: 1 by the following 3 amino acid substitutions: N2Y, K13E, and K152R. The amino acid substitutions are also evident from Figure 4. Furthermore, Figure 4 provides an alignment of the nucleic acid molecule encoding SEQ ID NO: 1, 3, and 5. SEQ ID NO: 2 encodes SEQ ID NO: 1 (mutant M12), SEQ ID NO: 4 encodes SEQ ID NO: 3 (wild-type GOx from *Aspergillus niger*) and SEQ ID NO: 6 encodes SEQ ID NO: 5 (parent mutant B11).

In this regard, the term "mutation" or "variation" means any detectable change in genetic material, e.g. DNA, or any process, mechanism, or result of such a change. This includes gene mutations, in which the structure (e.g. DNA sequence) of a gene is altered, any gene or DNA arising from any mutation process, and any expression product (e.g. protein or polynucleotide) expressed by a modified gene or DNA sequence. Such changes also include changes in the promoter, ribosome binding site, etc. "Sequence-conservative variations" of a polynucleotide sequence are those in which a change of one or more nucleotides in a given codon position results in no alteration in the amino acid encoded at that position.

In a preferred embodiment of the invention in (I)(c) and (II) as defined herein above at least three of (i) to (v) apply: (i) tyrosine is present in the amino acid position corresponding to amino acid position 2 of SEQ ID NO: 1, (ii) glutamic acid is present in the amino acid position corresponding to amino acid position 13 3 of SEQ ID NO: 1, (iii) valine is present in the amino acid position corresponding to amino acid position 30 of SEQ ID NO: 1, (iv) valine is present in the amino acid position corresponding to amino acid position 94 of SEQ ID NO: 1, and (v) arginine is present in the amino acid position corresponding to amino acid position 152 of SEQ ID NO: 1; and/or in (I)(d) as defined herein above at least three of (i) to (v) apply: (i) nucleotide positions 4 to 6 of SEQ ID NO: 2 code for tyrosine, (ii) nucleotide positions 26 to 28 of SEQ ID NO: 2 code for glutamic acid, (iii) nucleotide positions 90 to 92 of SEQ ID NO: 2 code for valine, (iv) nucleotide positions 282 to 284 of SEQ ID NO: 2 code for valine, and (v) nucleotide positions 456 to 458 of SEQ ID NO: 2 code for arginine.

As mentioned above and as it can be seen in Figure 4, SEQ ID NO. 3 of the invention differs from the mutant or variant having SEQ ID NO: 1 by the amino acid mutations N2Y, K13E, T30V, I94V, and K152R. Moreover, as it can be seen from Table 1, this mutations result in a novel GOx variant having improved enzymatic efficiency as compared to the parent GOx variant and the wild-type GOx. Accordingly, it preferred with increased preference that the nucleic acid molecule of the invention is characterized by at least three, at least four, at least five, or all of , K13E, T30V, 194V, and K152R.

In a further embodiment the invention relates to a vector encoding the nucleic acid molecule of the invention.

The term "vector" in accordance with the invention means preferably a plasmid, cosmid, virus, bacteriophage or another vector used e.g. conventionally in genetic engineering which carries the nucleic acid molecule of the invention either encoding the peptide or the fusion protein of the invention. Accordingly, the nucleic acid molecule of the invention may be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Invitrogen).

The nucleic acid molecules inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the polypeptide/protein or fusion protein of the invention is operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include neomycin, ampicillin, and hygromycine, kanamycin resistance and the like. Specifically-designed vectors allow the shuttling of DNA between different hosts, such as bacteria-fungal cells or bacteria-animal cells (e. g. the Gateway® system available at lnvitrogen). An expression vector according to this invention is capable of directing the replication, and the expression, of the polynucleotide and encoded peptide or fusion protein of this invention. Apart from introduction via vectors such as phage vectors or viral vectors (e.g. adenoviral, retroviral), the nucleic acid molecules as described herein above may be designed for direct introduction or for introduction via liposomes into a cell. Additionally, baculoviral systems or systems based on vaccinia virus or Semliki Forest virus can be used as eukaryotic expression systems for the nucleic acid molecules of the invention.

Another embodiment of the invention relates to a host cell transformed, transduced or transfected with the vector of the invention.

The term "host cell" means any cell of any organism that is selected, modified, transformed, grown, or used or manipulated in any way, for the production of a substance by the cell, for example the expression by the cell of a gene, a DNA or RNA sequence, a protein or an enzyme.

The host cell of the invention may be produced by introducing the nucleic acid molecule or vector(s) of the invention into the host cell which upon its/their presence mediates the expression of the nucleic acid molecule of the invention encoding the protein of the invention. The host from which the host cell is derived or isolated may be any prokaryote or eukaryotic cell or organism, with the exception of human embryonic stem cells that have been derived directly by destruction of a human embryo..

Suitable prokaryotes (bacteria) useful as hosts for the invention are those generally used for cloning and/or expression like *E*. *coli* (e.g., *E coli* strains BL21, HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomyces* or *Bacillus subtilis.* Appropriate culture mediums and conditions for the above-described host cells are well known in the art.

A suitable eukaryotic host cell may be a vertebrate cell, an insect cell, a fungal/yeast cell, a nematode cell or a plant cell. The fungal/yeast cell may a *Saccharomyces cerevisiae* cell, *Pichia pastoris* cell or an *Aspergillus* cell. Preferred examples for host cell to be genetically engineered with the nucleic acid molecule or the vector(s) of the invention is a cell of yeast, *E*. *coli* and/or a species of the genus *Bacillus* (e.g., *B. subtilis*)*.* Most preferred the host cell is a yeast cell (e.g. S. cerevisiae).

In one further embodiment the invention relates to a method of producing a protein having the activity of a glucose oxidase (EC 1.1.3.4) comprising (a) culturing the host cell of the invention and (b) isolating the produced protein having the activity of a glucose oxidase.

The term "culturing" specifies the process by which host cells are grown under controlled conditions. These conditions may vary dependent on the host cell used. The skilled person is well aware of methods for establishing optimized culturing conditions. Moreover, methods for establishing, maintaining and manipulating a cell culture have been extensively described in the state of the art.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001, Molecular Cloning: A laboratory manual, 3rd ed, Cold Spring Harbor Laboratory Press, New York.

The term "produced protein" in accordance with the invention refers to the product of a process implying, that in the host cell a process can be induced by which information from a nucleic acid molecule encoding the polypeptide is used in the synthesis of the polypeptide of the invention. Several steps in this process may be modulated, including the transcription, RNA splicing, translation, and post-translational modification of the peptide of the invention by methods know in the art. Accordingly, such modulation may allow for control of the timing, location, and amount of peptide produced.

The invention furthermore relates to a protein having the activity of a glucose oxidase (EC 1.1.3.4) encoded by the nucleic acid molecule of the invention or produced by the method of the invention.

The protein of the invention may be generated by molecular cloning techniques well known in the art. Recombinant expression can be accomplished, for example, by using vectors and host cells as described herein above.

According to a preferred embodiment, the protein of the invention is a fusion protein.

A "fusion protein" according to the present invention contains at least one additional heterologous amino acid sequence. Often, but not necessarily, these additional sequences will be located at the N- or C-terminal end of the polypeptide. It may e.g. be convenient to initially express the polypeptide as a fusion protein from which the additional amino acid residues can be removed, e.g. by a proteinase capable of specifically trimming the fusion protein and releasing the polypeptide of the present invention.

Those at least one additional heterologous amino acid sequence of said fusion proteins, include amino acid sequences which confer desired properties such as modified/enhanced stability, modified/enhanced solubility and/or the ability of targeting one or more specific cell types. For example, fusion proteins with antibodies specific for cell surface markers or with antigen-recognizing fragments of said antibodies are envisaged. Accordingly, the fusion protein may consist of or comprise protein domains known to function in signal transduction and/or known to be involved in protein-protein interaction. Examples for such domains are Ankyrin repeats; arm, Bcl-homology, Bromo, CARD, CH, Chr, C1, C2, DD, DED, DH, EFh, ENTH, F-box, FHA, FYVE, GEL, GYF, hect, LIM, MH2, PDZ, PB1, PH, PTB, PX, RGS, RING, SAM, SC, SH2, SH3, SOCS, START, TIR, TPR, TRAF, tsnare, Tubby, UBA, VHS, W, WW, and 14-3-3 domains. Further information about these and other protein domains is available from the databases InterPro (http://www.ebi.ac.uk/interpro/, Mulder et al., 2003, Nucl. Acids. Res. 31: 315-318), Pfam (http://www.sanger.ac.uk/Software/Pfam/, Bateman et al., 2002, Nucleic Acids Research 30(1 ): 276-280) and SMART (hftp://smart.embl-heidelberg.de/, Letunic et al., 2002, Nucleic Acids Res. 30(1 ), 242-244).

It is preferred that the at least one additional heterologous amino acid sequence encodes a polypeptide having the activity of a peroxidase as defined herein below.

In a further embodiment, the invention relates to a composition comprising the nucleic acid of the invention, the vector of the invention, the host cell of the invention, and/or the protein of the invention, or combinations thereof .

The term "composition" as used herein refers to a composition comprising at least one of the nucleic acid of the invention, the vector of the invention, the host cell of the invention, and/or the protein of the invention, or combinations thereof which are also referred in the following collectively as compounds.

The protein of the invention having the activity of a glucose oxidase is useful in various compositions, including but not limited to a food composition, a pharmaceutical composition, a cosmetic composition or a diagnostic composition to preserve these compositions, in particular during storage or as active agent providing potent anti-microbial activity. As it is well known in the art, glucose oxidase can be used to remove residual glucose and oxygen in a composition in order to prolong their shelf life. Moreover, the hydrogen peroxide produced by glucose oxidase acts as a good bactericide, and can optionally be later removed using an enzyme, e.g. catalase, that coverts hydrogen peroxide to oxygen and water.

In closed systems (e.g. air-tight capsules, containers, or packages) glucose oxidase can be used to consume oxygen, thereby reducing the oxidation/reduction potential, which decreases the rate of growth or stops growth of aerobic organisms, such as e.g. Bacillus species, and oxidative organisms, such as Pseudomonas species (Jon J Kabara and Donald S Orth (1997), Cosmetic Science and Technology, Technology & Engineering, 16:274).

It is preferred that the composition of the invention further comprises one or more of: glucose, peroxidase, catalase and a buffer system (e.g. a citric acid buffer system).

Moreover, it is preferred that the composition of the invention is a anti-microbial composition.

In accordance with a preferred embodiment of the invention, the composition of the invention is a food composition, a pharmaceutical composition, a cosmetic composition or a diagnostic composition.

The term "food composition" ("nutritional formulation") as used herein describes a solid or liquid formulation which can be eaten or drunk by a human or animal subject for nutrition. A food composition is distinguished from a vaccine. In contrast to a vaccine, a nutritional formulation does not comprise any of adjuvants (unless as contaminations), activated or inactivated viral compounds (unless as contaminations), activated or inactivated bacterial compounds (unless as contaminations), and pathogenic compounds (unless as contaminations). The food composition may further comprise supplements. The term "supplement" as used herein relates to a nutritional supplement which is a concentrated source of nutrient or alternatively other substances with a nutritional or physiological effect whose purpose is to supplement the normal diet.

In addition to the above recited ingredients, further ingredients may be selected from lipids, minerals, carbohydrates, amino acids, amino acid chelates, anabolic nutrients, vitamins, antioxidants, probiotic bacteria and lipotropic agents in order to provide an optimal sustained energy and anabolic nutritional formulation. The nutritional formulation may be a nutritional supplement or may provide complete nutrition. Preferably the nutritional formulation is in the form of a liquid.

In accordance with the present invention, the term "pharmaceutical composition" relates to a composition for administration to a patient, preferably a human patient. The pharmaceutical composition is preferably an anti-microbial composition or a anti-proliferative composition. A anti-proliferative composition may be used to teat any disease associated with cellular overgrowth, like benign of malignant tumours. The anti-proliferative effect of glucose oxidase, in particular of antibodies coupled to GOx used in combination with antibodies coupled to a peroxidase, is described in Stanislawski et al. (1989), Cancer Research 49; 5497-5504.

The pharmaceutical compositions described herein can be administered to the subject at a suitable or effective dose. Administration of the suitable or effective compositions may be effected by different ways (cf. below).

The pharmaceutical composition for use in accordance with the present invention can be formulated in a conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The pharmaceutical composition may, accordingly, be administered orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

The pharmaceutical composition of the invention can be formulated as neutral or salt forms.

Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The pharmaceutical composition of the invention can also, if desired, be presented in a pack, or dispenser device which can contain one or more unit dosage forms containing the said agent. The pack can for example comprise metal or plastic foil, such as blister pack. The pack or dispenser device can be accompanied with instruction for administration.

The pharmaceutical composition of the invention can be administered as sole active agent or can be administered in combination with other agents. It is preferred that it is administered in combination with a peroxidase or substance comprising a peroxidase.

The pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Examples of such carrier vehicles include water, saline, Ringer's solution, and dextrose solution. Non-aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and may include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

A "cosmetic composition" according to the invention is for use in non-therapeutic applications.

Cosmetic compositions may also be defined by their intended use, as compositions intended to be rubbed, poured, sprinkled, or sprayed on, or otherwise applied to the human body for cleansing, beautifying, promoting attractiveness, or altering the appearance.

The particular formulation of the cosmetic composition according to the invention is not limited. Envisaged formulations include rinse solutions, emulsions, creams, milks, gels such as hydrogels, ointments, suspensions, dispersions, powders, solid sticks, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613). Preferred formulations for said cosmetic composition are rinse solutions and creams.

The application of the composition of the invention in cosmetics is, inter alia, aiming at improving or maintaining the appearance of the skin and hair. Due to the anti-microbial activity of the protein of the invention, it can be used in cosmetic compositions in order to prevent or treat e.g. acne, dysfunction of sebaceous glands, inflammation or irritations of the skin, or dry skin. A further potential application of cosmetic compositions comprising the composition of the invention is the conditioning of hair, Furthermore, a use of GOx as antioxidant is conceivable. As a further application the peroxide generated by GOx could be used as active ingredient for pharmaceutical and cosmetic compositions to achieve antimicrobial effects.

A suitable concentration of the compound(s) of the invention for cosmetic use is considered to be in the range of 0,0001 to 1% (w/v), preferably 0,0001 to 0,1% (w/v), even more preferably 0,001 to 0,1 % (w/v).

Preferred amounts of the cosmetic compositions according to the invention to be applied in a single application are between 0.1 and 10g, more preferred between 0.1 and 1g, most preferred 0.5g. The amount to be applied also depends on the size of the area to be treated and has to be adapted thereto.

The term "peroxidase" (EC number 1.11.1.x) in accordance with the invention specifies an enzyme that catalyzes a reaction of the form ROOR' + electron donor (2 e-) + 2H⁺ → ROH + R'OH, wherein one substrate is hydrogen peroxide.

Glucose oxidase can be used for the analysis of industrial samples using quantification of peroxide to determine the amount of oxidized glucose e. g. for quality control of glucose containing products.

The term "industrial sample" as used herein means a sample of a solid, gel, or liquid which is intended for any industrial purpose (e.g. used in the process of the manufacture of food, a cosmetic, medicament, or diagnostic composition). It is preferred that the industrial sample is a liquid industrial sample. Non-limiting examples of liquid industrial samples are beverages, e.g. soft drinks or alcoholic drinks, fermentation liquids, syrups, mixtures, creams, ointments.

In summary, a novel variant of glucose oxidase with improved kinetic properties was developed via directed evolution and flow cytometry sorting.

The Figures show:
**Figure 1**: Scheme of the screening procedure starting with reaction in primary emulsion.
Figure 2: *S.cerevisiae* cells labeled with fluorescein tyramide after reaction in primary emulsion. The picture was recorded with LSM 510 Meta inverted laser scanning microscope.
Figure 3: Flow cytometer recordings of referent libraries with different percentages of cells expressing GOx activity. a) yeast cells with empty vector, b) 10% of GOx expressing cells, c) 100% GOx expressing cells, d) epPCR library. Cells were emulsified and stained with fluorescein tyramide according to GOx activity. Forward scattering (FSC) represents cell size and FL1-FITC represents green fluorescence signal.
Figure 4: Sequence alignment of the amino acid sequences encoding the wild-type glucose oxidase of Aspergillus niger (SEQ ID NO: 3) indicated as wt (top row), the parent glucose oxidase (SEQ ID NO: 5) indicated as having T30V-194V (middle row), and the glucose oxidase variant of the invention M12 (SEQ ID NO: 1) indicated as having N2Y-K13E-T30V-194V-K152R (lower row).

The examples illustrate the invention.

### General procedures and materials

### Materials

All chemicals used were of analytical reagent grade or higher quality and purchased from Sigma-Aldrich Chemie (Taufkirchen, Germany) and Applichem (Darmstadt, Germany). pYES2 shuttle vector, Escherichia coli strain DH5a, and *Saccharomyces cerevisiae* strain INVScI were purchased from Invitrogen (Karlsruhe, Germany). Nucleotides and all other enzymes were purchased from Fermentas (St. Leon-Rot, Germany), if not stated otherwise. A thermal cycler (Mastercycler gradient; Eppendorf, Hamburg, Germany) and thin-wall PCR tubes (Muiti-Uitra tubes; 0.2 mL; Carl Roth, Germany) were used for all PCRs and thermostability measurements. The PCR volume was always 50 pL; larger volumes were prepared in multiple 50-µL PCRs. The amount of DNA was quantified using a NanoDrop photometer (NanoDrop Technologies, Germany). Fluorescence micrographs were obtained using the LSM 510 Meta inverted laser scanning microscope (Carl Zeiss GmbH,Oberkochen, Germany) and stored in 1024 x 1024 pixel resolution as 8-bit LSM format. Excitation wavelengths were 488 nm (Argon laser), the pinhole was always set to one airy unit. Image analysis was performed with Zeiss LSM Image Browser version 3.2.0.70. (Carl Zeiss GmbH, Jena, Germany). Flow cytometry analysis and sorting was done using Partec CyFlow®ML (Munster, Germany) flow cytomer. For emulsification MICCRA D-1 dispenser (ART Prozess- & Labortechnik Gmbh & Co. KG, Müllheim) was used.

### Synthesis of fluorescein tyramide

Synthesis was done according to the published protocol [vanGijlswijk, R.P.M., et al., Fluorochrome-labeled tyramides: Use in immunocytochemistry and fluorescence in situ hybridization. Journal of Histochemistry & Cytochemistry, 1997. 45(3): p. 375-382]. Solution of tyramine HCI (mM), TEA (mM) and NHS-fluorescein (mM) in DMF was incubated 2h (4°C). After dilution with ethanol fluorescein tyramide was stored in the dark at 4°C until further use.

### epPCR library

For generating the mutant library the PCR reaction (94 °C for 4min, 1 cycle; 94 °C for 1 min/55 °C for 1min/72 °C for 2.3 min, 30 cycles; 72 °C for 10 min, 1 cycle) contained *Taq* DNA polymerase (0.04 U/µL), dNTP mix (0.2 mM), forward primer (0.5 µM) (5'-CATCGGGGTACCATGAGATTTCCTTC-3'), reverse primer (5'-GAAGCTCTAGAGCTCACTGCATGG-3') (0.5 µM), pYES2 plasmid B11 GOx (50 ng) and MnCl₂ (0.1 mM). The purified fragments were restriction digested and ligated in to the pYES2 (Invitrogen) vector. The ligation mixture was directly transformed into the S. *cerevisiae* competent cells, using the Gietz protocol [Gietz, R.D. and R.H. Schiestl, High-efficiency yeast transformation using the LiAc/SS carrier DNA/PEG method. Nature Protocols, 2007. 2(1): p. 31-34].

### Expression of the enzyme

After transformation, *S. cerevisiae* cells were grown for 24h at 30°C under agitation in liquid SCU-Glucose media [Zhu, Z., et al., Directed evolution of glucose oxidase from Aspergillus niger for ferrocenemethanol-mediated electron transfer. Biotechnology Journal, 2007. 2(2): p. 241-248], centrifuged (3000 rpm, 25°C), resuspended in SCU-galactose media and incubated for 4h at 30°C under agitation. Before compartmentalization cells were washed 3 times in PBS buffer pH 7.4.

### In vitro compartmentalization

A modification of a protocol which has been described before [Aharoni, A., et al., High-throughput screening of enzyme libraries: Thiolactonases evolved by fluorescence-activated sorting of single cells in emulsion compartments. Chemistry & Biology, 2005. 12(12): p. 1281-1289] was used for *in vitro* compartmentalization of yeast cells. 10⁷ cells were pelleted, resuspended in 80µL of the Tris buffer (50 mM, pH 7.4) containing fluorescein-tyramide (20 pM), glucose (100 mM), HRP (10 U/mL) and ascorbic acid (0.25 mM) and emulsified in 0.8 mL of 1.5% ABIL EM90 in light mineral oil (LMO) with MICCRA D-1 dispenser (8000 rpm, 3 min, on ice). The primary emulsion was incubated on ice for 30 minutes and then the reaction was stopped by stirring (5 min, 12000 rpm) with 100 pL of ascorbic acid (0.5 M), BSA (10 mg/mL) water solution.

### Recovery of cells

Cells were recovered from emulsion by stirring (5 min, 1200 rpm) with 1 mL of LMO containing Span 80 (3 %). Emulsion was centrifuged (2min, 14000 g), supernatant discarded and cells rinsed 3 times with LMO containing Span 80 (3 %), 2 times with PBS containing Triton X-100 (0.5 %) and finally with PBS.

### Example 1: Flow cytometry screening

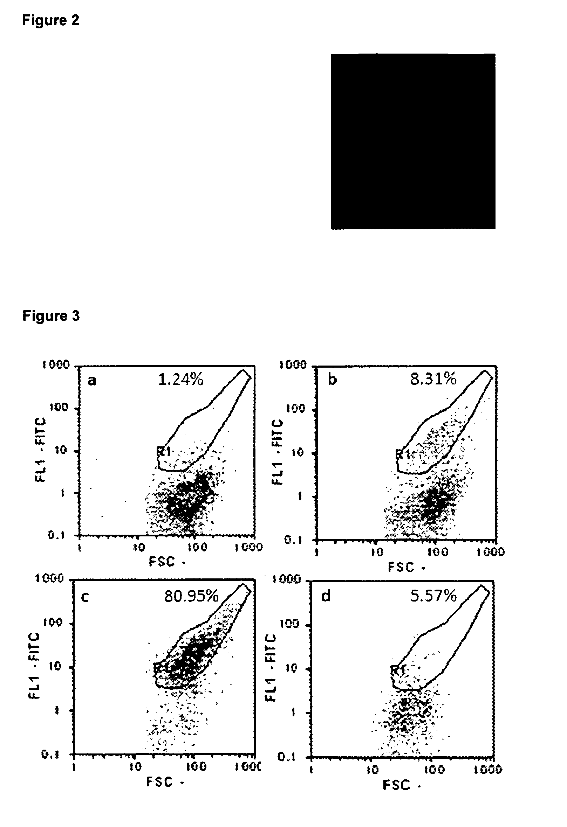

An epPCR mutant library of GOx with a ratio of 1 to 200 active to non-active was screened (10' cells; ~10⁵ muteins, Fig. 3 d). In order to validate the 5.57 % of active GOx population, 10 % of sorted cells were plated plated on SCU-Glucose agar plates, grown under non-inducing conditions to avoid a bias between the ratio of active to non-active variants, and subsequently replicated on SCU-Galactose inducing agar plates. Active variants were determined by an overlay agar assay using ABTS. 30 % of the sorted GOx variants were active and enrichment factor of 60 was achieved. In total 10⁷ cells were screened and thirty sorted GOx variants with high agar plate activity were selected for detailed analysis using a reported ABTS screening system in microtiter plates [Zhu, Z.W., et al., Making glucose oxidase fit for biofuel cell applications by directed protein evolution. Biosensors & Bioelectronics, 2006. 21(11): p. 2046-2051].

The cells were diluted in sheat fluid (0.9 % NaCl, 0.01 Triton X-100) and analyzed using Partec CyFlow®ML (Münster, Germany) flow cytometer. The trigger parameter was set on forward scattering. The analysis rate was around 4000 events/s and the sorting speed was 10-100/s. The blue solid state 200 mW laser with an excitation at 488 nm and a 100 W UV lamp with 366 nm (200-600 nm) were used for analysis. The emission was detected with the 520 nm filter. The positive emulsion droplets were gated on the FL1-FITC, FL3 -DAPI plot. The sorted cells were centrifuged, plated and grown on SCU-Glucose agar plates (2 days, 30 °C). GOx was induced by replica plating and growing of cells on SCU-Galactose plates (1 day, 30°C). The cells were overlayed with ABTS agar containing low melting agarose (2 %), ABTS (3.3 mM), HRP (5 U/mL) and glucose (333 mM) in PBS. The positive cells were picked, grown in MTP and the activity was determined using the ABTS liquid assay. Mutants that showed the highest activity were purified. The plasmid DNA was isolated from yeast according to a previously described protocol [Holm, C., et al., A Rapid, Efficient Method For Isolating DNA From Yeast. Gene, 1986. 42(2): p. 169-173.24], retransformed in *E. coli* for high production, isolated again and sent for sequencing.

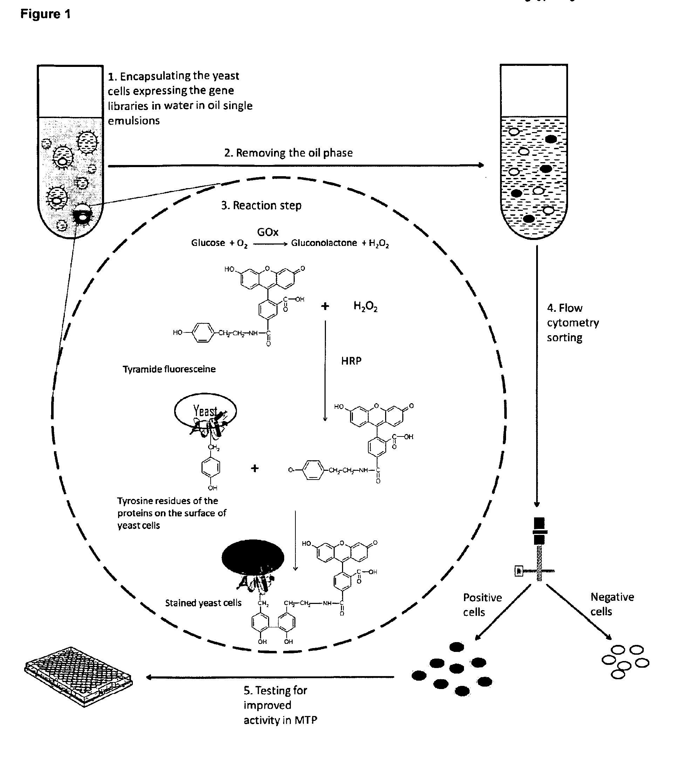

The amount of GOx activity located in the surrounding media and attached and/or entrapped on the S. *cerevisiae* cell wall/membrane was quantified (fig. 1, fig. 2). Interestingly, up to 50 % of GOx expressed for 12 h of fermentation stayed attached to S. *cerevisiae* after washing.

Based on these recordings gate array for sorting was carefully selected considering the results from Fig. 3 (a-c) using for normalization the region where the cells are located expressing active GOx. After sorting, enrichment factors of 4.7 times were achieved for ratio of 1 to 10, 13 times for ratio 1 to 100 and 33 times for ratio 1 to 1000. In all referent libraries we obtained after sorting an increase in percentage of cells expressing GOx, and the enrichment factor was higher for libraries with lower percentage of positive cells. These factors prove that the applied screening technology can be efficiently used to enrich by flow cytometer cell populations with active GOx.

### Example 2: Isolation of mutant GOx

The wild type GOx (GenBank accession no. X16061, Swiss-Prot: P13006), the parent mutant (T30V-194V) (Momeu, I. C., 2007, Improving glucose oxidase properties by directed evolution, Thesis (Ph.D.), International University Bremen, http://wwwback.jacobs-university.de/phd/files/1169800479.pdf) and the mutant M12 (SEQ ID NO: 2) were isolated from 0.5 L fermentation media. Cells were first grown in liquid SCU-Glucose media (48 h, 30°C, 250 rpm), centrifuged (5 min, 25°C, 3000 g), diluted in SCU-Galactose media (0.4 O.D.600nm), grown again (12h, 30°C, 250 rpm) and centrifuged again (5 min, 25°C, 3000 g). Supernatant was concentrated by ultrafiltration using 50 kDa cut-off membranes (Amicon) and equilibrated by dialysis (10 mM sodium phosphate buffer, pH 6.0). GOx was purified through chromatography on DEAE TSK 650S column (1.0 cm x 18 cm) equilibrated with the same buffer. The gradient program was as follows: washing with 50 ml of the equilibrating buffer; linear gradient (100 ml) of 0.01-1M sodium phosphate buffer pH 6.0. Fractions containing GOx activity were pooled dialyzed against distilled water and lyophilized. Purity of GOx was confirmed by SDS PAGE and UV-VIS spectroscopy.

### Example 3: Evaluation of kinetic parameters of novel glucose oxidases

The kinetic constants were determined using the Specord 200 from (Analytik Jena, Germany) and the ABTS assay [Zhu, Z.W., et al. 2006, Making glucose oxidase fit for biofuel cell applications by directed protein evolution. Biosensors & Bioelectronics, 21(11): 2046-2051]. The measurements were performed in 1 ml of reaction volume and 1 cm light pathway cuvettes, using 2.5, 5, 10, 25, 50 and 100 mM glucose. The data were fitted using Origin 6 (OriginLab Corporation, Northampton).

In total 10' cells were screened and thirty sorted GOx variants with high agar plate activity were selected for detailed analysis using a reported ABTS screening system in microtiter plates. The sequence of clone M12 was determined to identify the mutations leading to changed properties. The protein was purified to measure kinetic constants (Table 1).

The best mutant, termed M12, showed a slightly increased Kₘ and almost two times increased k_{c}at value compared to parent GOx and compared to the wild-type a 1.2 fold decrease in Km and 2.7 fold increase in k_{cat} The 87.5 % increase in k_{cat} of GOx M12 is beyond statistical error due to the high absolute activity of GOx for glucose. Thus, the enzymatic efficieny of the GOx of the invention was improved significantly.

**Table 1 Kinetic constants of mutant, parent and wt GOx**

| | k_{cat} | Kₘ | k_{cm}/Kₘ | Mutations |
|---|---|---|---|---|
| wt GOx | 54.8 s' | 22.0 mM | 2.49 mM/s | - |
| Parent GOx | 80.0 S-¹ | 16.0 mM | 5.00 mM/s | T30V, 194V |
| M12 | 150 s-1 | 18.1 mM | 8.26 mM/s | N2Y, K13E, T30V, 194V, K152R |

## Claims

1. A nucleic acid molecule encoding
(I) a polypeptide having the activity of a glucose oxidase (EC 1.1.3.4), which is
(a) a nucleic acid molecule encoding a polypeptide comprising or consisting of the amino acid sequence of SEQ ID NO: 1;
(b) a nucleic acid molecule comprising or consisting of the nucleotide sequence of SEQ ID NO: 2;
(c) a nucleic acid molecule encoding a glucose oxidase the amino acid sequence of which is at least 85 %, preferably at least 90 % identical, more preferably at least 95 % identical, and most preferred at least 98% identical to the amino acid sequence of SEQ ID NO: 1, provided that at least three of (i) to (v) apply:
(i) tyrosine, serine, threonine, cysteine, asparagine, glutamine, aspartic acid or glutamic acid is present in the amino acid position corresponding to amino acid position 2 of SEQ ID NO: 1,
(ii) glutamic acid, aspartic acid, tyrosine, serine, threonine, asparagine or glutamine is present in the amino acid position corresponding to amino acid position 13 of SEQ ID NO: 1,
(iii) valine, glycine, alanine, isoleucine, leucine, methionine, phenylalanine, tryptophan or proline is present in the amino acid position corresponding to amino acid position 30 of SEQ ID NO: 1,
(iv) valine, glycine, alanine, isoleucine, leucine, methionine, phenylalanine, tryptophan or proline is present in the amino acid position corresponding to amino acid position 94 of SEQ ID NO: 1, and
(v) arginine or histidine is present in the amino acid position corresponding to amino acid position 152 of SEQ ID NO: 1;
(d) a nucleic acid molecule comprising or consisting of a nucleotide sequence which is at least 85 % identical, preferably at least 90 % identical, more preferably at least 95 % identical, and most preferred at least 98 % identical to the nucleotide sequence of SEQ ID NO: 2; provided that at least three of (i) to (v) apply:
(i) nucleotide positions 4 to 6 of SEQ ID NO: 2 code for tyrosine, serine, threonine, cysteine, asparagine, glutamine, aspartic acid or glutamic acid,
(ii) nucleotide positions 26 to 28 of SEQ ID NO: 2 code for glutamic acid, aspartic acid, tyrosine, serine, threonine, asparagine or glutamine,
(iii) nucleotide positions 90 to 92 of SEQ ID NO: 2 code for valine, glycine, alanine, isoleucine, leucine, methionine, phenylalanine, tryptophan or proline,
(iv) nucleotide positions 282 to 284 of SEQ ID NO: 2 code for valine, glycine, alanine, isoleucine, leucine, methionine, phenylalanine, tryptophan or proline, and
(v) nucleotide positions 456 to 458 of SEQ ID NO: 2 code for arginine or histidine;
(e) a nucleic acid molecule which is degenerate with respect to the nucleic acid molecule of (d); or
(f) a nucleic acid molecule corresponding to the nucleic acid molecule of any one of (a) to (e) wherein T is replaced by U;
(II) a fragment of the polypeptide of (I) having the activity of a glucose oxidase and comprising at least 400 amino acids, provided that at least three of (i) to (v) apply
(i) tyrosine, serine, threonine, cysteine, asparagine, glutamine, aspartic acid or glutamic acid is present in the amino acid position corresponding to amino acid position 2 of SEQ ID NO: 1,
(ii) glutamic acid, aspartic acid, tyrosine, serine, threonine, asparagine or glutamine is present in the amino acid position corresponding to amino acid position 13 of SEQ ID NO: 1,
(iii) valine, glycine, alanine, isoleucine, leucine, methionine, phenylalanine, tryptophan or proline is present in the amino acid position corresponding to amino acid position 30 of SEQ ID NO: 1,
(iv) valine, glycine, alanine, isoleucine, leucine, methionine, phenylalanine, tryptophan or proline is present in the amino acid position corresponding to amino acid position 94 of SEQ ID NO: 1, and
(v) arginine or histidine is present in the amino acid position corresponding to amino acid position 152 of SEQ ID NO: 1.

2. The nucleic acid molecule of claim 1,
wherein in (I)(c) and (II) at least three of (i) to (v) apply:
(i) tyrosine is present in the amino acid position corresponding to amino acid position 2 of SEQ ID NO: 1,
(ii) glutamic acid is present in the amino acid position corresponding to amino acid position 13 of SEQ ID NO: 1,
(iii) valine is present in the amino acid position corresponding to amino acid position 30 of SEQ ID NO: 1,
(iv) valine is present in the amino acid position corresponding to amino acid position 94 of SEQ ID NO: 1, and
(v) arginine is present in the amino acid position corresponding to amino acid position 152 of SEQ ID NO: 1; and/or
wherein in (I)(d) at least three of (i) to (v) apply:
(i) nucleotide positions 4 to 6 of SEQ ID NO: 2 code for tyrosine,
(ii) nucleotide positions 26 to 28 of SEQ ID NO: 2 code for glutamic acid,
(iii) nucleotide positions 90 to 92 of SEQ ID NO: 2 code for valine,
(iv) nucleotide positions 282 to 284 of SEQ ID NO: 2 code for valine, and
(v) nucleotide positions 456 to 458 of SEQ ID NO: 2 code for arginine.

3. A vector encoding the nucleic acid molecule of claim 1 or 2.

4. A host cell transformed, transduced or transfected with the vector of claim 3.

5. A method of producing a protein having the activity of a glucose oxidase (EC 1.1.3.4) comprising
(a) culturing the host cell of claim 4 and
(b) isolating the produced protein having the activity of a glucose oxidase.

6. A protein having the activity of a glucose oxidase (EC 1.1.3.4) encoded by the nucleic acid molecule of claim 1 or 2 or produced by the method of claim 5.

7. The protein of claim 6 which is a fusion protein.

8. A composition comprising the nucleic acid of claim 1 or 2, the vector of claim 3, the host cell of claim 4, and/or the protein of claim 6 or 7, or combinations thereof.

9. The composition of claim 8, which is a food composition, a pharmaceutical composition, a diagnostic composition or a cosmetic composition.


SEQUENCE LISTING

<110>  B.R.A.I.N. AG

<120>  A NOVEL GLUCOSE OXIDASE

<130>  R2708 EP

<160>  6

<170>  PatentIn version 3.3

<210>  1
<211>  583
<212>  PRT
<213>  Aspergillus niger

<400>  1

Ser Tyr Gly Ile Glu Ala Ser Leu Leu Thr Asp Pro Glu Asp Val Ser
1               5                   10                  15

Gly Arg Thr Val Asp Tyr Ile Ile Ala Gly Gly Gly Leu Val Gly Leu
            20                  25                  30

Thr Thr Ala Ala Arg Leu Thr Glu Asn Pro Asn Ile Ser Val Leu Val
        35                  40                  45

Ile Glu Ser Gly Ser Tyr Glu Ser Asp Arg Gly Pro Ile Ile Glu Asp
    50                  55                  60

Leu Asn Ala Tyr Gly Asp Ile Phe Gly Ser Ser Val Asp His Ala Tyr
65                  70                  75                  80

Glu Thr Val Glu Leu Ala Thr Asn Asn Gln Thr Ala Leu Val Arg Ser
                85                  90                  95

Gly Asn Gly Leu Gly Gly Ser Thr Leu Val Asn Gly Gly Thr Trp Thr
            100                 105                 110

Arg Pro His Lys Ala Gln Val Asp Ser Trp Glu Thr Val Phe Gly Asn
        115                 120                 125

Glu Gly Trp Asn Trp Asp Asn Val Ala Ala Tyr Ser Leu Gln Ala Glu
    130                 135                 140

Arg Ala Arg Ala Pro Asn Ala Arg Gln Ile Ala Ala Gly His Tyr Phe
145                 150                 155                 160

Asn Ala Ser Cys His Gly Val Asn Gly Thr Val His Ala Gly Pro Arg
                165                 170                 175

Asp Thr Gly Asp Asp Tyr Ser Pro Ile Val Lys Ala Leu Met Ser Ala
            180                 185                 190

Val Glu Asp Arg Gly Val Pro Thr Lys Lys Asp Phe Gly Cys Gly Asp

195                 200                 205

Pro His Gly Val Ser Met Phe Pro Asn Thr Leu His Glu Asp Gln Val
    210                 215                 220

Arg Ser Asp Ala Ala Arg Glu Trp Leu Leu Pro Asn Tyr Gln Arg Pro
225                 230                 235                 240

Asn Leu Gln Val Leu Thr Gly Gln Tyr Val Gly Lys Val Leu Leu Ser
                245                 250                 255

Gln Asn Gly Thr Thr Pro Arg Ala Val Gly Val Glu Phe Gly Thr His
            260                 265                 270

Lys Gly Asn Thr His Asn Val Tyr Ala Lys His Glu Val Leu Leu Ala
        275                 280                 285

Ala Gly Ser Ala Val Ser Pro Thr Ile Leu Glu Tyr Ser Gly Ile Gly
    290                 295                 300

Met Lys Ser Ile Leu Glu Pro Leu Gly Ile Asp Thr Val Val Asp Leu
305                 310                 315                 320

Pro Val Gly Leu Asn Leu Gln Asp Gln Thr Thr Ala Thr Val Arg Ser
                325                 330                 335

Arg Ile Thr Ser Ala Gly Ala Gly Gln Gly Gln Ala Ala Trp Phe Ala
            340                 345                 350

Thr Phe Asn Glu Thr Phe Gly Asp Tyr Ser Glu Lys Ala His Glu Leu
        355                 360                 365

Leu Asn Thr Lys Leu Glu Gln Trp Ala Glu Glu Ala Val Ala Arg Gly
    370                 375                 380

Gly Phe His Asn Thr Thr Ala Leu Leu Ile Gln Tyr Glu Asn Tyr Arg
385                 390                 395                 400

Asp Trp Ile Val Asn His Asn Val Ala Tyr Ser Glu Leu Phe Leu Asp
                405                 410                 415

Thr Ala Gly Val Ala Ser Phe Asp Val Trp Asp Leu Leu Pro Phe Thr
            420                 425                 430

Arg Gly Tyr Val His Ile Leu Asp Lys Asp Pro Tyr Leu His His Phe
        435                 440                 445

Ala Tyr Asp Pro Gln Tyr Phe Leu Asn Glu Leu Asp Leu Leu Gly Gln
    450                 455                 460

Ala Ala Ala Thr Gln Leu Ala Arg Asn Ile Ser Asn Ser Gly Ala Met

465                 470                 475                 480

Gln Thr Tyr Phe Ala Gly Glu Thr Ile Pro Gly Asp Asn Leu Ala Tyr
                485                 490                 495

Asp Ala Asp Leu Ser Ala Trp Thr Glu Tyr Ile Pro Tyr His Phe Arg
            500                 505                 510

Pro Asn Tyr His Gly Val Gly Thr Cys Ser Met Met Pro Lys Glu Met
        515                 520                 525

Gly Gly Val Val Asp Asn Ala Ala Arg Val Tyr Gly Val Gln Gly Leu
    530                 535                 540

Arg Val Ile Asp Gly Ser Ile Pro Pro Thr Gln Met Ser Ser His Val
545                 550                 555                 560

Met Thr Val Phe Tyr Ala Met Ala Leu Lys Ile Ser Asp Ala Ile Leu
                565                 570                 575

Glu Asp Tyr Ala Ser Met Gln
            580

<210>  2
<211>  1752
<212>  DNA
<213>  Aspergillus niger

<400>  2
agctatggca ttgaggccag cctcctgact gatcccgagg atgtctccgg ccgcacggtc      60

gactacatca tcgctggtgg aggtctggtt ggactcacca ccgctgctcg tctgacggag     120

aaccccaaca tcagtgtgct cgtcatcgaa agtggctcct acgagtcgga cagaggtcct     180

atcattgagg acctgaacgc ctacggcgac atctttggca gcagtgtaga ccacgcctac     240

gagaccgtgg agctcgctac caacaatcaa accgcgctgg tccgctccgg aaatggtctc     300

ggtggctcta ctctagtgaa tggtggcacc tggactcgcc cccacaaggc acaggttgac     360

tcttgggaga ctgtctttgg aaatgagggc tggaactggg acaatgtggc cgcctactcc     420

ctccaggctg agcgtgctcg cgcaccaaat gccagacaga tcgctgctgg ccactacttc     480

aacgcatcct gccatggtgt taatggtact gtccatgccg gaccccgcga caccggcgat     540

gactattctc ccatcgtcaa ggctctcatg agcgctgtcg aagaccgggg cgttcccacc     600

aagaaagact tcggatgcgg tgacccccat ggtgtgtcca tgttccccaa caccttgcac     660

gaagaccaag tgcgctccga tgccgctcgc gaatggctac ttcccaacta ccaacgtccc     720

aacctgcaag tcctgaccgg acagtatgtt ggtaaggtgc tccttagcca gaacggcacc     780

acccctcgtg ccgttggcgt ggaattcggc acccacaagg gcaacaccca caacgtttac     840

gctaagcacg aggtcctcct ggccgcgggc tccgctgtct ctcccacaat cctcgaatat     900

tccggtatcg gaatgaagtc catcctggag ccccttggta tcgacaccgt cgttgacctg     960

cccgtcggct tgaacctgca ggaccagacc accgctaccg tccgctcccg catcacctct 1020

gctggtgcag gacagggaca ggccgcttgg ttcgccacct tcaacgagac ctttggtgac 1080

tattccgaaa aggcacacga gctgctcaac accaagctgg agcagtgggc cgaagaggcc 1140

gtcgcccgtg gcggattcca caacaccacc gccttgctca tccagtacga gaactaccgc 1200

gactggattg tcaaccacaa cgtcgcgtac tcggaactct tcctcgacac tgccggagta 1260

gccagcttcg atgtgtggga ccttctgccc ttcacccgag gatacgttca catcctcgac 1320

aaggacccct accttcacca cttcgcctac gaccctcagt acttcctcaa cgagctggac 1380

ctgctcggtc aggctgccgc tactcaactg gcccgcaaca tctccaactc cggtgccatg 1440

cagacctact tcgctgggga gactatcccc ggtgataacc tcgcgtatga tgccgatttg 1500

agcgcctgga ctgagtacat cccgtaccac ttccgtccta actaccatgg cgtgggtact 1560

tgctccatga tgccgaagga gatgggcggt gttgttgata atgctgcccg tgtgtatggt 1620

gtgcagggac tgcgtgtcat tgatggttct attcctccta cgcaaatgtc gtcccatgtc 1680

atgacggtgt tctatgccat ggcgctaaaa atttcggatg ctatcttgga agattatgct 1740

tccatgcagt ga 1752

<210> 3
<211> 583
<212> PRT
<213> Aspergillus niger

<400> 3

Ser Asn Gly Ile Glu Ala Ser Leu Leu Thr Asp Pro Lys Asp Val Ser
1               5                   10                  15

Gly Arg Thr Val Asp Tyr Ile Ile Ala Gly Gly Gly Leu Thr Gly Leu
            20                  25                  30

Thr Thr Ala Ala Arg Leu Thr Glu Asn Pro Asn Ile Ser Val Leu Val
        35                  40                  45

Ile Glu Ser Gly Ser Tyr Glu Ser Asp Arg Gly Pro Ile Ile Glu Asp
    50                  55                  60

Leu Asn Ala Tyr Gly Asp Ile Phe Gly Ser Ser Val Asp His Ala Tyr
65                  70                  75                  80

Glu Thr Val Glu Leu Ala Thr Asn Asn Gln Thr Ala Leu Ile Arg Ser
                85                  90                  95

Gly Asn Gly Leu Gly Gly Ser Thr Leu Val Asn Gly Gly Thr Trp Thr
            100                 105                 110

Arg Pro His Lys Ala Gln Val Asp Ser Trp Glu Thr Val Phe Gly Asn
        115                 120                 125

Glu Gly Trp Asn Trp Asp Asn Val Ala Ala Tyr Ser Leu Gln Ala Glu
    130                 135                 140

Arg Ala Arg Ala Pro Asn Ala Lys Gln Ile Ala Ala Gly His Tyr Phe
145                 150                 155                 160

Asn Ala Ser Cys His Gly Val Asn Gly Thr Val His Ala Gly Pro Arg
                165                 170                 175

Asp Thr Gly Asp Asp Tyr Ser Pro Ile Val Lys Ala Leu Met Ser Ala
            180                 185                 190

Val Glu Asp Arg Gly Val Pro Thr Lys Lys Asp Phe Gly Cys Gly Asp
        195                 200                 205

Pro His Gly Val Ser Met Phe Pro Asn Thr Leu His Glu Asp Gln Val
    210                 215                 220

Arg Ser Asp Ala Ala Arg Glu Trp Leu Leu Pro Asn Tyr Gln Arg Pro
225                 230                 235                 240

Asn Leu Gln Val Leu Thr Gly Gln Tyr Val Gly Lys Val Leu Leu Ser
                245                 250                 255

Gln Asn Gly Thr Thr Pro Arg Ala Val Gly Val Glu Phe Gly Thr His
            260                 265                 270

Lys Gly Asn Thr His Asn Val Tyr Ala Lys His Glu Val Leu Leu Ala
        275                 280                 285

Ala Gly Ser Ala Val Ser Pro Thr Ile Leu Glu Tyr Ser Gly Ile Gly
    290                 295                 300

Met Lys Ser Ile Leu Glu Pro Leu Gly Ile Asp Thr Val Val Asp Leu
305                 310                 315                 320

Pro Val Gly Leu Asn Leu Gln Asp Gln Thr Thr Ala Thr Val Arg Ser
                325                 330                 335

Arg Ile Thr Ser Ala Gly Ala Gly Gln Gly Gln Ala Ala Trp Phe Ala
            340                 345                 350

Thr Phe Asn Glu Thr Phe Gly Asp Tyr Ser Glu Lys Ala His Glu Leu
        355                 360                 365

Leu Asn Thr Lys Leu Glu Gln Trp Ala Glu Glu Ala Val Ala Arg Gly
    370                 375                 380

Gly Phe His Asn Thr Thr Ala Leu Leu Ile Gln Tyr Glu Asn Tyr Arg
385                 390                 395                 400

Asp Trp Ile Val Asn His Asn Val Ala Tyr Ser Glu Leu Phe Leu Asp
                405                 410                 415

Thr Ala Gly Val Ala Ser Phe Asp Val Trp Asp Leu Leu Pro Phe Thr
            420                 425                 430

Arg Gly Tyr Val His Ile Leu Asp Lys Asp Pro Tyr Leu His His Phe
        435                 440                 445

Ala Tyr Asp Pro Gln Tyr Phe Leu Asn Glu Leu Asp Leu Leu Gly Gln
    450                 455                 460

Ala Ala Ala Thr Gln Leu Ala Arg Asn Ile Ser Asn Ser Gly Ala Met
465                 470                 475                 480

Gln Thr Tyr Phe Ala Gly Glu Thr Ile Pro Gly Asp Asn Leu Ala Tyr
                485                 490                 495

Asp Ala Asp Leu Ser Ala Trp Thr Glu Tyr Ile Pro Tyr His Phe Arg
            500                 505                 510

Pro Asn Tyr His Gly Val Gly Thr Cys Ser Met Met Pro Lys Glu Met
        515                 520                 525

Gly Gly Val Val Asp Asn Ala Ala Arg Val Tyr Gly Val Gln Gly Leu
    530                 535                 540

Arg Val Ile Asp Gly Ser Ile Pro Pro Thr Gln Met Ser Ser His Val
545                 550                 555                 560

Met Thr Val Phe Tyr Ala Met Ala Leu Lys Ile Ser Asp Ala Ile Leu
                565                 570                 575

Glu Asp Tyr Ala Ser Met Gln
            580

<210>  4
<211>  1752
<212>  DNA
<213>  Aspergillus niger

<400>  4
agcaatggca ttgaagccag cctcctgact gatcccaagg atgtctccgg ccgcacggtc      60

gactacatca tcgctggtgg aggtctgact ggactcacca ccgctgctcg tctgacggag     120

aaccccaaca tcagtgtgct cgtcatcgaa agtggctcct acgagtcgga cagaggtcct     180

atcattgagg acctgaacgc ctacggcgac atctttggca gcagtgtaga ccacgcctac     240

gagaccgtgg agctcgctac caacaatcaa accgcgctga tccgctccgg aaatggtctc     300

ggtggctcta ctctagtgaa tggtggcacc tggactcgcc cccacaaggc acaggttgac     360

tcttgggaga ctgtctttgg aaatgagggc tggaactggg acaatgtggc cgcctactcc     420

ctccaggctg agcgtgctcg cgcaccaaat gccaaacaga tcgctgctgg ccactacttc 480

aacgcatcct gccatggtgt taatggtact gtccatgccg gaccccgcga caccggcgat 540

gactattctc ccatcgtcaa ggctctcatg agcgctgtcg aagaccgggg cgttcccacc 600

aagaaagact tcggatgcgg tgacccccat ggtgtgtcca tgttccccaa caccttgcac 660

gaagaccaag tgcgctccga tgccgctcgc gaatggctac ttcccaacta ccaacgtccc 720

aacctgcaag tcctgaccgg acagtatgtt ggtaaggtgc tccttagcca gaacggcacc 780

acccctcgtg ccgttggcgt ggaattcggc acccacaagg gcaacaccca caacgtttac 840

gctaagcacg aggtcctcct ggccgcgggc tccgctgtct ctcccacaat cctcgaatat 900

tccggtatcg gaatgaagtc catcctggag ccccttggta tcgacaccgt cgttgacctg 960

cccgtcggct tgaacctgca ggaccagacc accgctaccg tccgctcccg catcacctct 1020

gctggtgcag gacagggaca ggccgcttgg ttcgccacct tcaacgagac ctttggtgac 1080

tattccgaaa aggcacacga gctgctcaac accaagctgg agcagtgggc cgaagaggcc 1140

gtcgcccgtg gcggattcca caacaccacc gccttgctca tccagtacga gaactaccgc 1200

gactggattg tcaaccacaa cgtcgcgtac tcggaactct tcctcgacac tgccggagta 1260

gccagcttcg atgtgtggga ccttctgccc ttcacccgag gatacgttca catcctcgac 1320

aaggacccct accttcacca cttcgcctac gaccctcagt acttcctcaa cgagctggac 1380

ctgctcggtc aggctgccgc tactcaactg gcccgcaaca tctccaactc cggtgccatg 1440

cagacctact tcgctgggga gactatcccc ggtgataacc tcgcgtatga tgccgatttg 1500

agcgcctgga ctgagtacat cccgtaccac ttccgtccta actaccatgg cgtgggtact 1560

tgctccatga tgccgaagga gatgggcggt gttgttgata atgctgcccg tgtgtatggt 1620

gtgcagggac tgcgtgtcat tgatggttct attcctccta cgcaaatgtc gtcccatgtc 1680

atgacggtgt tctatgccat ggcgctaaaa atttcggatg ctatcttgga agattatgct 1740

tccatgcagt ga 1752

<210> 5
<211> 583
<212> PRT
<213> Aspergillus niger

<400> 5

Ser Asn Gly Ile Glu Ala Ser Leu Leu Thr Asp Pro Lys Asp Val Ser
1               5                   10                  15

Gly Arg Thr Val Asp Tyr Ile Ile Ala Gly Gly Gly Leu Val Gly Leu
            20                  25                  30

Thr Thr Ala Ala Arg Leu Thr Glu Asn Pro Asn Ile Ser Val Leu Val
        35                  40                  45

Ile Glu Ser Gly Ser Tyr Glu Ser Asp Arg Gly Pro Ile Ile Glu Asp

50                  55                  60

Leu Asn Ala Tyr Gly Asp Ile Phe Gly Ser Ser Val Asp His Ala Tyr
65                  70                  75                  80

Glu Thr Val Glu Leu Ala Thr Asn Asn Gln Thr Ala Leu Val Arg Ser
                85                  90                  95

Gly Asn Gly Leu Gly Gly Ser Thr Leu Val Asn Gly Gly Thr Trp Thr
            100                 105                 110

Arg Pro His Lys Ala Gln Val Asp Ser Trp Glu Thr Val Phe Gly Asn
        115                 120                 125

Glu Gly Trp Asn Trp Asp Asn Val Ala Ala Tyr Ser Leu Gln Ala Glu
    130                 135                 140

Arg Ala Arg Ala Pro Asn Ala Lys Gln Ile Ala Ala Gly His Tyr Phe
145                 150                 155                 160

Asn Ala Ser Cys His Gly Val Asn Gly Thr Val His Ala Gly Pro Arg
                165                 170                 175

Asp Thr Gly Asp Asp Tyr Ser Pro Ile Val Lys Ala Leu Met Ser Ala
            180                 185                 190

Val Glu Asp Arg Gly Val Pro Thr Lys Lys Asp Phe Gly Cys Gly Asp
        195                 200                 205

Pro His Gly Val Ser Met Phe Pro Asn Thr Leu His Glu Asp Gln Val
    210                 215                 220

Arg Ser Asp Ala Ala Arg Glu Trp Leu Leu Pro Asn Tyr Gln Arg Pro
225                 230                 235                 240

Asn Leu Gln Val Leu Thr Gly Gln Tyr Val Gly Lys Val Leu Leu Ser
                245                 250                 255

Gln Asn Gly Thr Thr Pro Arg Ala Val Gly Val Glu Phe Gly Thr His
            260                 265                 270

Lys Gly Asn Thr His Asn Val Tyr Ala Lys His Glu Val Leu Leu Ala
        275                 280                 285

Ala Gly Ser Ala Val Ser Pro Thr Ile Leu Glu Tyr Ser Gly Ile Gly
    290                 295                 300

Met Lys Ser Ile Leu Glu Pro Leu Gly Ile Asp Thr Val Val Asp Leu
305                 310                 315                 320

Pro Val Gly Leu Asn Leu Gln Asp Gln Thr Thr Ala Thr Val Arg Ser

325                 330                 335

Arg Ile Thr Ser Ala Gly Ala Gly Gln Gly Gln Ala Ala Trp Phe Ala
            340                 345                 350

Thr Phe Asn Glu Thr Phe Gly Asp Tyr Ser Glu Lys Ala His Glu Leu
        355                 360                 365

Leu Asn Thr Lys Leu Glu Gln Trp Ala Glu Glu Ala Val Ala Arg Gly
    370                 375                 380

Gly Phe His Asn Thr Thr Ala Leu Leu Ile Gln Tyr Glu Asn Tyr Arg
385                 390                 395                 400

Asp Trp Ile Val Asn His Asn Val Ala Tyr Ser Glu Leu Phe Leu Asp
                405                 410                 415

Thr Ala Gly Val Ala Ser Phe Asp Val Trp Asp Leu Leu Pro Phe Thr
            420                 425                 430

Arg Gly Tyr Val His Ile Leu Asp Lys Asp Pro Tyr Leu His His Phe
        435                 440                 445

Ala Tyr Asp Pro Gln Tyr Phe Leu Asn Glu Leu Asp Leu Leu Gly Gln
    450                 455                 460

Ala Ala Ala Thr Gln Leu Ala Arg Asn Ile Ser Asn Ser Gly Ala Met
465                 470                 475                 480

Gln Thr Tyr Phe Ala Gly Glu Thr Ile Pro Gly Asp Asn Leu Ala Tyr
                485                 490                 495

Asp Ala Asp Leu Ser Ala Trp Thr Glu Tyr Ile Pro Tyr His Phe Arg
            500                 505                 510

Pro Asn Tyr His Gly Val Gly Thr Cys Ser Met Met Pro Lys Glu Met
        515                 520                 525

Gly Gly Val Val Asp Asn Ala Ala Arg Val Tyr Gly Val Gln Gly Leu
    530                 535                 540

Arg Val Ile Asp Gly Ser Ile Pro Pro Thr Gln Met Ser Ser His Val
545                 550                 555                 560

Met Thr Val Phe Tyr Ala Met Ala Leu Lys Ile Ser Asp Ala Ile Leu
                565                 570                 575

Glu Asp Tyr Ala Ser Met Gln
            580

<210>  6

<211>  1752
<212>  DNA
<213>  Aspergillus niger

<400>  6
agcaatggca ttgaagccag cctcctgact gatcccaagg atgtctccgg ccgcacggtc      60

gactacatca tcgctggtgg aggtctggtt ggactcacca ccgctgctcg tctgacggag     120

aaccccaaca tcagtgtgct cgtcatcgaa agtggctcct acgagtcgga cagaggtcct     180

atcattgagg acctgaacgc ctacggcgac atctttggca gcagtgtaga ccacgcctac     240

gagaccgtgg agctcgctac caacaatcaa accgcgctgg tccgctccgg aaatggtctc     300

ggtggctcta ctctagtgaa tggtggcacc tggactcgcc cccacaaggc acaggttgac     360

tcttgggaga ctgtctttgg aaatgagggc tggaactggg acaatgtggc cgcctactcc     420

ctccaggctg agcgtgctcg cgcaccaaat gccaaacaga tcgctgctgg ccactacttc     480

aacgcatcct gccatggtgt taatggtact gtccatgccg gaccccgcga caccggcgat     540

gactattctc ccatcgtcaa ggctctcatg agcgctgtcg aagaccgggg cgttcccacc     600

aagaaagact tcggatgcgg tgacccccat ggtgtgtcca tgttccccaa caccttgcac     660

gaagaccaag tgcgctccga tgccgctcgc gaatggctac ttcccaacta ccaacgtccc     720

aacctgcaag tcctgaccgg acagtatgtt ggtaaggtgc tccttagcca gaacggcacc     780

acccctcgtg ccgttggcgt ggaattcggc acccacaagg gcaacaccca caacgtttac     840

gctaagcacg aggtcctcct ggccgcgggc tccgctgtct ctcccacaat cctcgaatat     900

tccggtatcg gaatgaagtc catcctggag ccccttggta tcgacaccgt cgttgacctg     960

cccgtcggct tgaacctgca ggaccagacc accgctaccg tccgctcccg catcacctct    1020

gctggtgcag gacagggaca ggccgcttgg ttcgccacct tcaacgagac ctttggtgac    1080

tattccgaaa aggcacacga gctgctcaac accaagctgg agcagtgggc cgaagaggcc    1140

gtcgcccgtg gcggattcca caacaccacc gccttgctca tccagtacga gaactaccgc    1200

gactggattg tcaaccacaa cgtcgcgtac tcggaactct tcctcgacac tgccggagta    1260

gccagcttcg atgtgtggga ccttctgccc ttcacccgag gatacgttca catcctcgac    1320

aaggacccct accttcacca cttcgcctac gaccctcagt acttcctcaa cgagctggac    1380

ctgctcggtc aggctgccgc tactcaactg gcccgcaaca tctccaactc cggtgccatg    1440

cagacctact tcgctgggga gactatcccc ggtgataacc tcgcgtatga tgccgatttg    1500

agcgcctgga ctgagtacat cccgtaccac ttccgtccta actaccatgg cgtgggtact    1560

tgctccatga tgccgaagga gatgggcggt gttgttgata atgctgcccg tgtgtatggt    1620

gtgcagggac tgcgtgtcat tgatggttct attcctccta cgcaaatgtc gtcccatgtc    1680

atgacggtgt tctatgccat ggcgctaaaa atttcggatg ctatcttgga agattatgct    1740

tccatgcagt ga                                                        1752